# EUROPEAN PATENT APPLICATION

(11) **EP 3 428 276 A1**
(43) Date of publication of application: **16.01.2019**
(21) Application number: 16893681.3
(22) Date of filing: 09.08.2016
(51) Int. Cl.: C12N 13/00, C12M 1/42, C12M 1/36, C12N 5/077, C12N 5/071, C12N 5/074

(54) **CELL REPROGRAMMING APPARATUS**

(30) Priority: 11.03.2016 KR 20160029707; 03.08.2016 KR 20160098754
(71) Applicant: STEMON Inc., Gyeonggi-do 13483 (KR)
(72) Inventor: KIM, Soon Hag, Seongnam-si Gyeonggi-do 13589 (KR)
(74) Representative: Zardi, Marco
(86) International application number: PCT/KR2016/008755
(87) International publication number: WO 2017/155167

(57) **Abstract**

The present invention relates to a cell reprogramming apparatus using energy capable of promoting an environmental influx, and more particularly, the cell reprogramming apparatus has an effect of inducing reprogramming into new types of pluripotent cells having pluripotent characteristics or arbitrary differentiated cells having a different expression type from the differentiated cells by applying energy such as ultrasonic waves, laser, or heat treatment to the differentiated cells.

## Description

### [Technical Field]

The present invention relates to an apparatus and a method for cell reprogramming, which are capable of promoting an environmental influx through ultrasonic waves, laser, heat treatment, etc. The national research and development project that supports the present invention is a high-tech medical technology development project sponsored by the Ministry of Health and Welfare. The project has a unique number "HI14C3297", titled "Development of stem cell distribution and neural differentiation monitoring method *in vivo* using microRNA tracing system in ischemic brain injury model" and is supported by the Catholic Kwandong University Industry Cooperation Foundation which is a managing department. In addition, the national research and development project supporting the present invention is a researcher support project supported by the Ministry of Science, ICT and Future Planning, and has a unique number "2013R1A2A2A01068140", titled "Development of microRNA-based stem cell differentiation tracking radiation biomolecule imaging method", and is supported by the Catholic Kwandong University Industry Cooperation Foundation which is a managing department.

### [Background Art]

A method for reprogramming somatic cells into other types of cells, progenitor cells, and stem cells is a clinically important technique in cell therapy, disease models, and transplantation. These techniques have been currently attempted through molecular and chemical methods targeting several pluripotent genes, a variety of differentiation-specific expression genes, and the like. However, existing methods are pointed out in terms of stability and efficiency, and have a disadvantage of a complicated process.

Cells are exposed to a variety of environments, which influence the gene expression of the cells in a short or long time as the cells pass through the generation, and a gene expression program of cells is regulated by stress due to an environmental change. In a cell culture environment, a medium ingredient contains various substances and ions, and the intracellular influx of such an environment may be a revolutionary method to promote cell change. However, the cells are not well transduced and infused due to the degree of polarity and size of the various components of the cell culture medium due to the cell membrane composed of phospholipids. Recently, it has been reported that the microbubbles and a cavitation effect generated by the ultrasonic wave cause the intracellular influx of the external environment, and the ultrasonic wave stimulation has a positive effect on the cell development. It is also reported that ATP is induced by ultrasonic waves and such ATP reacts with receptors of the cell membrane to induce substance transport.

In this respect, the inventors of the present invention have contrived a method of delivering various substances into cells by temporarily damaging the somatic cell membrane using physical stimulation such as an ultrasonic wave and utilizing a cavitation effect of the medium due to the ultrasonic wave. The inventors completed the present invention by developing a cell reprogramming method using a physical stimulation-mediated environmental influx, a so-called "physical stimulation-mediated permeation of Environmental transition guided cellular reprogramming, ENTER cells".

### [Summary of Invention]

### [Technical Problem]

An object of the present invention is to provide a cell reprogramming apparatus capable of promoting an environmental influx through ultrasonic waves, laser, heat treatment, etc., for differentiated cells.

### [Solution to Problem]

In order to solve the object, according to an aspect of the present invention, there is provided a cell reprogramming apparatus including: a culture chamber provided to accommodate cells and a culture medium; and one or more devices provided to provide energy by applying at least one physical stimulation of ultrasonic waves, laser, and heat to the cells and the culture medium in the culture chamber.

The mechanism may include at least one of an ultrasonic generation device for irradiating the ultrasonic waves, a laser irradiation device for irradiating the laser, and a temperature control device.

The cell reprogramming apparatus may further include at least one of a humidity control device for controlling humidity in the culture chamber and a gas control device for controlling the carbon dioxide amount in the culture chamber.

The cell reprogramming apparatus may further include a display unit having an input unit for controlling at least one of a temperature, the humidity, and the carbon dioxide amount in the culture chamber.

The mechanism may include the ultrasonic generation device, and the display unit may be provided so as to set an ultrasonic frequency and a time.

A sample tube accommodating the cells may be disposed in the culture chamber, and the ultrasonic generation device may be elevatably provided toward the sample tube.

A sample tube holder for supporting the sample tube may be provided in the culture chamber.

The sample tube may have a dual tube structure.

In the ultrasonic generation device, an ultrasonic transducer may be replaceably provided.

A temperature control device may include a heat wire provided around the culture chamber.

According to another aspect of the present invention, there is provided a cell reprogramming apparatus including: an auto sampler provided such that a plurality of sample tubes may be mounted; and an ultrasonic generation device provided to generate ultrasonic waves to any one sample tube mounted on the auto sampler.

The auto sampler may be rotatably provided, and the ultrasonic generation device may be elevatably provided.

When the auto sampler rotates, the ultrasonic generation device may be in an elevated state.

The cell reprogramming apparatus may further include a culture medium tank storing an culture medium treated with ultrasonic waves; one or more culture flasks to which the culture medium in the culture medium tank is supplied; and an injection tube connecting the culture flask to the sample tube for fluid flow.

Cells in the sample tube to which the ultrasonic waves are applied may be provided to move to the culture flask by the injection tube.

The cell reprogramming apparatus may further include a moving belt for transporting a plurality of culture flasks.

The cell reprogramming apparatus may further include: an incubator accommodating the culture flask; a recovery tank recovering the culture medium of the culture flask in the incubator; and a circulator for supplying the culture medium to the culture flask in the incubator and recovering the culture medium in the culture flask to the recovery tank.

Flow passages may be provided at both sides of the culture flask, respectively.

The flow passages at both sides of the culture flask may be positioned at different heights.

A cap may be mounted on each flow passage.

At least one cap may be provided so that an injector for culture medium flow is mounted.

A shaker for shaking the culture flasks may be provided in the incubator.

### [Advantageous Effects of Invention]

As described above, the cell reprogramming apparatus according to an embodiment of the present invention has the following effects.

The cell reprogramming apparatus has an effect of inducing reprogramming into new types of pluripotent cells having pluripotent characteristics or arbitrary differentiated cells having a different expression type from the differentiated cells by applying energy such as ultrasonic waves, laser, or heat treatment to the differentiated cells.

### [Brief Description of Drawings]

FIGS. 1 and 2 are conceptual diagrams illustrating a cell reprogramming apparatus according to a first embodiment of the present invention.
FIG. 3 is a conceptual diagram showing various embodiments of an ultrasonic transducer constituting the cell reprogramming apparatus shown in FIG. 1.
FIG. 4 is a conceptual diagram illustrating a sample tube constituting the cell reprogramming apparatus shown in FIG. 1.
FIGS. 5 to 7 are conceptual diagrams illustrating a cell reprogramming apparatus according to a second embodiment of the present invention.
FIG. 8 is a diagram illustrating a culture flask constituting the cell reprogramming apparatus shown in FIG. 5.
FIG. 9 is a diagram illustrating an injector mounted on the culture flask.
FIG. 10 is a diagram illustrating a state in which the culture flask is mounted on the injector.

### [Description of Embodiments]

Hereinafter, a cell reprogramming apparatus according to an exemplary embodiment of the present invention will be described in detail with reference to the accompanying drawings.

Further, the same or corresponding constituent elements are denoted by the same or similar reference numeral and a duplicated description thereof will be omitted and for easy description, the size and shape of each constituent member illustrated may be enlarged or reduced.

The present invention provides a cell reprogramming method including subjecting a mixture of differentiated cells and a culture medium to physical stimulation which can promote an environmental influx, and culturing the mixture subjected to the physical stimulation for a predetermined time to obtain reprogrammed cells.

The present invention is characterized in that the differentiated cells are cultured in any medium capable of inducing desired reprogrammed cells while subjecting differentiated cells to physical stimulation which can promote an environmental influx such as ultrasonic waves, laser, heat treatment, etc. to induce reprogramming of cells into pluripotent cells; or arbitrary differentiated cells having a different expression type from the differentiated cells, for example, hepatocytes, osteoblasts, adipocytes, myocytes, neurons, astrocytes, keratinocytes, hair follicle cells, pancreatic beta cells or cardiomyocytes.

For example, if pluripotent cells are intended as reprogrammed cells, the differentiated cells may be reprogrammed into pluripotent cells by mixing the differentiated cells with a stem cell culture medium and culturing the mixture for a predetermined time by subjecting the mixture to physical stimulation.

As another example, when arbitrary differentiated cells having an expression type different from that of the differentiated cells are intended as the reprogrammed cells, the differentiated cells may be reprogrammed into arbitrary differentiated cells having a different expression type by mixing the differentiated cells with a differentiation-inducing medium of desired differentiated cells and culturing the mixture for a predetermined time by subjecting the mixture to physical stimulation.

In the cell reprogramming method of the present invention, the reprogramming of the differentiated cells is induced according to an environmental influx other than the cells through physical stimulation to the differentiated cells. Such an environmental influx means an influx into the adjacent differentiated cells of genetic materials, chemicals, small molecules, or exosomes released from the differentiated cells subjected to the physical stimulation; or culture medium components.

According to the cell reprogramming method of the present invention, the environmental influx into the differentiated cells may determine reprogramming directivity into pluripotent cells stably expressing a pluripotent marker or a triploblastic marker and differentiated cells having a different expression type from the differentiated cells.

In addition, the reprogramming directivity may be determined by a kind of culture medium.

That is, as described above, the reprogramming from the differentiated cells to the pluripotent cells may be induced by subjecting the mixture of the differentiated cells and the stem cell culture medium to the physical stimulation, and the reprogramming from the differentiated cells into arbitrary differentiated cells having a different expression type may be induced by subjecting the mixture of the differentiated cells and the differentiation-inducing culture medium of the arbitrary differentiated cells to the physical stimulation.

With regard to the environmental influx into the differentiated cells, the present inventors have particularly considered cell membrane damage by physical stimulation and cellular secretion materials (exosomes). That is, the ultrasonic waves, laser, heat shock, etc. induce temperature rise by energy, oscillation of microbubbles generated by ultrasonic waves, and induction of liquid flow generation, that is, generation of microstream along the cell membrane to apply minute damage to the cell membrane due to such an effect and induce generation of holes so that absorption of external materials is increased. It is confirmed that in a change of cytosol Ca²⁺ concentration, that is, analysis of a change of cytosol Ca²⁺ concentration, when the damage to the cell membrane or cell membrane fluidity is increased, a cytosol Ca²⁺ concentration is instantaneously increased and thus the cell membrane fluidity is increased. According to one embodiment of the present invention, it can be seen that the Ca²⁺ concentration immediately after ultrasonic wave treatment is rapidly increased and then gradually decreased to be decreased to a level of a non-sonicated control group and restored after the damage to the cell membrane is induced. It is also known that ATP generation and increase due to ultrasonic waves induce response on various cellular stresses and endocytosis by reacting with ATP receptors in the cell membrane. In other words, there is a relation between ATP concentration and cell damage and intracellular substance influx, and in order to verify the relation, as a result of analyzing ATP concentration in cells after ultrasonic wave treatment, the ATP concentration was higher than that in the untreated control group. In addition, expression of ionic P2X receptors and metabolic P2Y receptors in ATP-affected cell membranes is also activated in the cells treated with ultrasonic waves compared to the control group. These results indicate the possibility of influx of extracellular environment as well as intracellular damage by ultrasonic waves.

Meanwhile, it is known that the exosomes include genetic information materials (DNA, mRNA, microRNA, protein) therein, and when the exosomes released outside the cell membrane through the cell membrane damage enter other neighboring cells again, the genetic information materials in the exosomes may be delivered. Accordingly, due to ultrasonic waves stimulation, expression of pluripotent markers which have been maintained in a low expression state or expression-suppressed state in the cells is induced and promoted and simultaneously, the damage to the cell membrane occurs, and thus, the exosomes present in the cells including the pluripotent markers of which the expression is induced or promoted are released outside to be delivered to the neighboring cells. Since the neighboring cells are also in a state where the cell membrane is partially damaged, the cell membrane fluidity is increased and thus it is estimated that the efficiency in which the exosomes enter the inside of the cells is higher than that in a normal state, and it is considered that the expression-induced and promoted pluripotency-related genetic information present in the exosomes is delivered so that pluripotent cells are produced. In one embodiment of the present invention, during a pluripotent cell inducing process, the culture medium is recovered, the exosomes in the medium are extracted, and then it is confirmed whether the pluripotent cell-related pluripotent markers are present therein, and as a result, it is confirmed that known pluripotent markers exhibit a high expression degree and thus it is considered that the hypothesis of the present inventors is supported. In addition, it has also been shown that even in ultrasonic waves, laser, or heat shock, the exosomes or the extracellular vesicles containing exosomes are normal without malformation of karyotypes.

This hypothesis makes it possible to produce pluripotent cells by inducing the release of exosomes due to cell membrane damage.

As the differentiated cells, somatic cells including mammalian-derived dermal fibroblasts, skin fibroblasts, and the like; cancer cells including uterine cancer cells (HeLa), liver cancer cells (Hep3B), and the like; or endotracheal cells including pulmonary epithelial cells (L132 cells), and the like may be used.

In this specification, the term "somatic cell" refers to cells constituting an adult and having limited differentiation potency and autopoiesis. According to one embodiment, the somatic cells may be somatic cells constituting the skin, hair, and fat of a mammal, preferably, mammalian-derived fibroblasts, but are not limited thereto.

In this specification, the term "pluripotent cells" refer to cells having pluripotency after physical stimulation, strictly, ultrasonic waves, laser, or heat treatment. In this specification, the pluripotency refers to a state in which pluripotent markers expressed in stem cells comprehensively are stably expressed. In addition, the pluripotency refers to a state in which triploblastic markers of endoderm, ectoderm, and mesoderm are expressed. The pluripotent cells may be used as "embryonic stem cell media-based environmental transition-guided cellular reprogramming (es/ENTER) cells.

The pluripotent cells according to the present invention are differentiated from known induced pluripotent stem cells in that the differentiation is induced well according to an external environment and a property of progenitor cell having a higher differentiation property than the property of a stem cell is higher. That is, when embryonic stem cells such as induced pluripotent stem cells are used as a cell therapeutic agent, a preparation step is required to undergo a certain degree of differentiation, and a risk factor that can be transformed into cancer is implicated, and a safety problem from using viral vectors to introduce a reprogramming inducing factor is raised. However, since the pluripotent cells of the present invention are induced without introducing a reprogramming inducing factor for genetic mutation or a reprogramming inducing substance such as a chemical material, culture through co-culture with different types of cells is not required, and thus, there is no cell contamination (problem of mixing with other cells) problem, and there is no problem in cancer generation without forming teratoma similar to cancer cells in an in-vivo experiment, thereby ensuring safety. In other words, the pluripotent cells of the present invention have an advantage that the induction process is simple and short, and the time for transplantation may be drastically shortened by treating autologous cells.

The pluripotent cell is characterized to stably express a pluripotent marker of any one of OCT3/4, S0X2, NANOG, c-MYC, KLF4, TDGF1, SSEA4, TRA-1-60, PAX6, Nestin, Brachyury, SMA, GATA4, or AFP or a triploblastic marker gene consisting of mesoderm or endoderm.

In this specification, the term "reprogramming" means a process of restoring or converting differentiated cells present in different types such as cells having no differentiation potency or cells having partial differentiation potency to final new type of cells or a state having new type of differentiation potency. According to the present invention, when the differentiated cells are subjected to the physical stimulation which can promote the environmental influx, the differentiated cells may be reprogrammed to pluripotent cells or desired arbitrary differentiated cells having an expression type different from differentiated cells.

Examples of the differentiated cells may include neurons (referred to as "neuronal stem cell media-based ENTER, n/ENTER") expressing any one of PAX6, Nestin, MAP2, TuJ1, GFAP, or O4; myocytes (referred to as "muscle differentiation media-based ENTER, m/ENTER") expressing any one of Desmin, Actinin, SMA, GATA4, or NKX2-5; hepatocytes (referred to as "hepatocyte differentiation media-based ENTER, h/ENTER") expressing any one of AFP, HNF4a, CK18, or ALB, and adipocytes (referred to as "adipocyte differentiation media-based ENTER, a/ENTER") expressing any one of Pparc2, C/ebpa, aP2, or Fabp4, but are not limited thereto.

In this specification, the "culture medium" is a medium used for cell culture in vitro in a comprehensive sense, and in the present invention, the "culture medium" means a stem cell culture medium or a differentiation-inducing medium, and the stem cell culture medium more particularly means an embryonic stem cell culture medium. In addition, the "differentiation-inducing medium" is a medium used for induction to differentiated cells of general stem cells, and for example, may be a hepatocyte differentiation-inducing medium, an osteogenic differentiation-inducing medium, an adipocyte differentiation-inducing medium, a myocyte differentiation-inducing medium, an astrocyte differentiation-inducing medium, a neuronal cell differentiation-inducing medium, a keratinocyte differentiation-inducing medium, a pancreatic beta cell differentiation-inducing medium, a cardiomyocyte differentiation-inducing medium, or the like, but is not limited thereto.

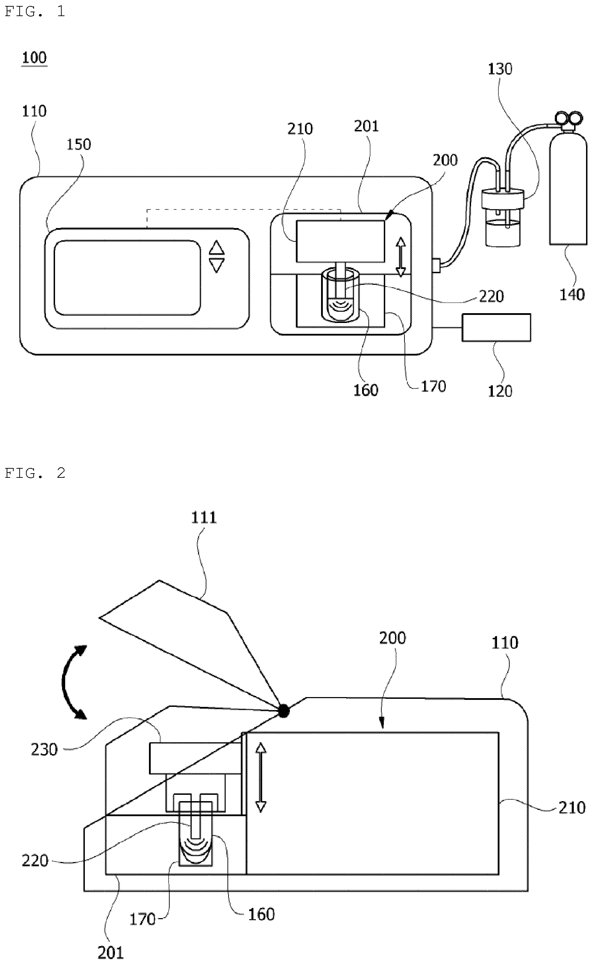

The cell reprogramming method of the present invention will be described in detail with reference to FIG. 1.

First, the culture medium is mixed with differentiated cells, and the mixture is subjected to the physical stimulation.

The reprogramming efficiency of the cells may be enhanced by subjecting the culture medium to the physical stimulation before subjecting the mixture including the differentiated cells to the physical stimulation.

The physical stimulation may be any one of ultrasonic waves, laser, or heat treatment.

The ultrasonic wave treatment for the culture medium may be performed by applying ultrasonic waves having an output intensity of 1 W/cm² to 20 W/cm² for 1 to 20 minutes, specifically ultrasonic waves having an output intensity of 2 W/cm² to 10 W/cm² for 5 to 15 minutes, and more specifically ultrasonic waves having an output intensity of 3 W/cm² to 7 W/cm² for 7 to 13 minutes.

The laser treatment for the culture medium may be performed by irradiating a pulsed laser beam with a wavelength band of 300 to 900 nm for 1 minute to 20 minutes, more specifically the pulsed laser beam with the wavelength band for 3 minutes to 15 minutes, and much more specifically the pulsed laser beam with the wavelength band for 5 to 10 minutes. The wavelength band may use, for example, wavelengths of 400 nm, 808 nm, and 880 nm.

The heat shock for the culture medium may be performed at a temperature of 40 to 50°C for 5 to 20 minutes.

When the differentiated cells are subjected to the physical stimulation, it is preferable to exposure the differentiated cells at a predetermined intensity, and a cell survival rate may be reduced out of the above range.

Accordingly, the ultrasonic wave treatment for the mixture of the culture medium and the differentiated cells may be performed by applying ultrasonic waves having an output intensity of 0.5 W/cm² to 3 W/cm² for 1 to 5 seconds, specifically ultrasonic waves having an output intensity of 0.7 W/cm² to 2 W/cm² for 1 to 5 seconds, and more specifically ultrasonic waves having an output intensity of 0.8 W/cm² to 1.5 W/cm² for 1 to 5 seconds.

The laser treatment for the mixture of the culture medium and the differentiated cells may be performed by irradiating a pulsed laser beam with a wavelength band of 300 to 900 nm for 1 second to 20 seconds, more specifically the pulsed laser beam with the wavelength band for 3 seconds to 10 seconds, and much more specifically the pulsed laser beam with the wavelength band for 4 to 6 seconds. The wavelength band may use, for example, wavelengths of 400 nm, 808 nm, and 880 nm.

The heat treatment for the mixture of the culture medium and the differentiated cells may be performed by exposure for 1 to 10 minutes at a temperature condition of 40 to 50°C and then exposure for 5 to 10 seconds at a temperature condition of 0 to 4°C.

Next, the mixture subjected to the physical stimulation is cultured for a predetermined time to obtain reprogrammed cells.

The culture of the mixture subjected to the physical stimulation may be performed for a period during which spheroid stably expressing the pluripotent marker or the differentiation marker is formed through a suspended culture or monolayer culture method, that is, for 2 to 10 days, but is not particularly limited thereto.

According to one embodiment of the present invention, the suspended culture exhibits efficiency of spheroid formation higher than that of the monolayer culture. In addition, the suspended culture has a larger number and size of spheroid than that of the monolayer culture and exhibits a constant size distribution.

According to one embodiment of the present invention, the expression of the pluripotent marker or the differentiation marker is increased or stabilized from about 3 days during the suspended culture of ultrasonic waves or laser-treated human skin fibroblasts, and reprogramming is started from this point. In addition, the expression of the pluripotent marker is increased or stabilized at about 8 days during the suspended culture of heat-treated human skin fibroblasts, and reprogramming is started from this period.

The pluripotency of the spheroid can be confirmed by expression of the pluripotent marker such as OCT3/4, SOX2, NANOG, c-MYC, KLF4, TDGF1, SSEA4, and TRA-1-60. The confirmation of the pluripotency marker may be analyzed through RT-PCR or immunocytochemistry, but is not particularly limited thereto.

In addition, the pluripotent cells of the present invention have a feature of a high level of expression of triploblastic markers, that is, ectodermal (PAX6, Nestin), mesenchymal (Brachyury, SMA), and endodermal (GATA4, AFP) markers.

In another embodiment, when the skin fibroblasts are subjected to the physical stimulation in the differentiation-inducing medium, the spheroid may be formed between about 2 to 6 days after the culture.

The differentiation marker may be at least one of PAX6, Nestin, MAP2, TuJl, GFAP, or O4 when reprogrammed into neurons.

The differentiation marker may be at least one of Desmin, Actinin, SMA, GATA4, or NKX2-5 when reprogrammed into myocytes.

The differentiation marker may be at least one of AFP, HNF4a, CK18, or ALB when reprogrammed into hepatocytes.

The differentiation marker may be stained with oil red O and may be at least one of Pparc2, C/ebpa, aP2, or Fabp4 when reprogrammed into adipocytes.

Further, the pluripotent cells of the present invention are characterized by having proliferation ability by expressing a proliferation marker protein, Ki-67.

In addition, when the reprogrammed pluripotent cells are co-cultured with nutritious cells, proliferation of the pluripotent cells may be increased.

Further, the cell reprogramming method of the present invention may further include culturing the pluripotent cells in the differentiation-inducing medium. Depending on a type of differentiation-inducing medium, the pluripotent cells may be differentiated into desired differentiated cells.

Examples of the differentiation-inducing medium may include a hepatocyte differentiation-inducing medium, an osteogenic differentiation-inducing medium, an adipocyte differentiation-inducing medium, a myocyte differentiation-inducing medium, an astrocyte differentiation-inducing medium, a neuronal cell differentiation-inducing medium, a keratinocyte differentiation-inducing medium, a pancreatic beta cell differentiation-inducing medium, a cardiomyocyte differentiation-inducing medium, or the like, but are not particularly limited thereto.

Hereinafter, a cell reprogramming apparatus capable of performing a cell reprogramming method described above will be described in detail with reference to the accompanying drawings.

As described above, a physical stimulation applied to cells may be any one of ultrasonic waves, laser, and heat treatment.

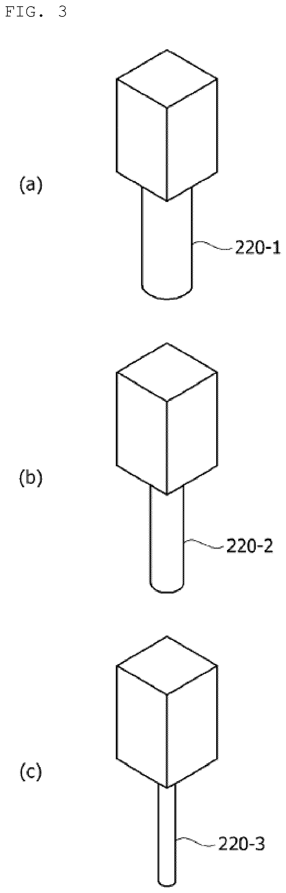

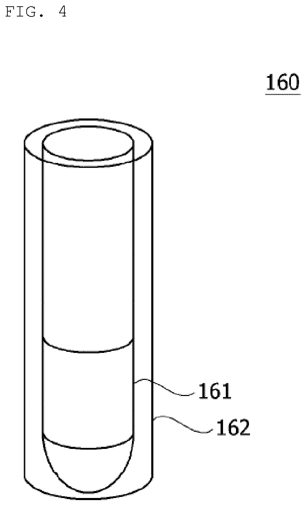

FIGS. 1 and 2 are conceptual diagrams illustrating a cell reprogramming apparatus 100 according to a first embodiment of the present invention, FIG. 3 is a conceptual diagram showing various embodiments of ultrasonic transducers 220-1, 220-2, and 220-3 constituting the cell reprogramming apparatus shown in FIG. 1, and FIG. 4 is a conceptual diagram illustrating a sample tube 160 constituting the cell reprogramming apparatus shown in FIG. 1.

Referring to FIGS. 1 and 2, the cell reprogramming apparatus 100 according to an embodiment of the present invention includes: a culture chamber 110 provided to accommodate cells and a culture medium; and one or more devices provided to provide energy by applying at least one physical stimulation of ultrasonic waves, laser, and heat to the cells and the culture medium in the culture chamber 110.

Further, the mechanism may include at least one of an ultrasonic generation device 200 for irradiating the ultrasonic waves, a laser irradiation device for irradiating the laser, and a temperature control device 120.

Further, the cell reprogramming apparatus 100 may further include at least one of a humidity control device 130 for controlling humidity in the culture chamber 110 and a gas control device 140 for controlling the carbon dioxide amount in the culture chamber 110.

In addition, the cell reprogramming apparatus 100 may further include a display unit 150 having an input unit for controlling at least one of temperature, humidity, and carbon dioxide amount in the culture chamber 110.

Further, when the mechanism is the ultrasonic generation device 200, the display unit 150 may be provided to set an ultrasonic frequency and a time.

Specifically, the present invention includes the culture chamber 110 capable of accommodating the cells and the culture medium and a mechanism capable of providing energy capable of promoting an environmental influx to the cells and the culture medium. Further, there is provided an induction device of a reprogrammed cell in the differentiated cell, wherein the reprogrammed cell is induced by providing the energy capable of promoting environmental influx to a mixture of the differentiated cell and the culture medium, and by culturing the mixture provided with the energy for a predetermined time.

The culture chamber 110 means a chamber which is generally usable in cell culturing. Further, the culture chamber 110 may be a chamber in which the temperature, the humidity, and/or gas are/is controllable so as to enable the cell culturing. For example, the culture chamber 110 includes a temperature control unit and carbon dioxide control unit, and a cell culture condition in the culture chamber 110 may be appropriately adjusted at levels of those skilled in the art according to a purpose and a type of cell.

Further, the culture chamber 110 may use a suspended culture or a single layer culture method of cells, and as a result, the culture chamber 110 may have a structure in which such a culture is available. For example, the culture chamber 110 may be a culture chamber equipped with an agitator for the suspended culture.

The apparatus capable of providing the energy capable of promoting the environmental influx may include an ultrasonic generation device 200 capable of irradiating the ultrasonic waves, a laser generation device (not shown) capable of irradiating the laser, or a temperature control device 120. Further, a function of the temperature control device 120 may be realized through a sample tube holder 170 to be described below.

When the ultrasonic generation device 200 is a known ultrasonic device that generates the ultrasonic waves having a frequency of 10 kHz to 100 MHz, the ultrasonic generation device may be used without a limit. The ultrasonic generation device may include an ultrasonic generator 210 and an ultrasonic transducer 220 (220-1, 220-2, and 220-3). Further, the ultrasonic generation device 200 may be provided so that the ultrasonic transducer 220 is replaceable. In addition, the ultrasonic generation device 200 may be provided in an ultrasonic chamber 201.

The laser generation device may adopt a laser device which generates a pulsed laser beam having a wavelength band of 300 to 900 nm, has a power of 1 to 15 W, has pulse duration of 1 to 900 ms, and has a frequency of 1 to 100 Hz, but is not particularly limited thereto.

The temperature control device 120 may adopt a known temperature control device capable of controlling a temperature of -40 to 99.0°C, but is not particularly limited thereto. For example, the temperature control device 120 may be a heat wire provided around the culture chamber.

The induction device of the reprogrammed cell in the differentiated cell of the present invention may induce reprogramming to the pluripotent cell or differentiated cell by performing the ultrasonic waves, the laser, or the heat treatment for the mixture of the culture medium and the differentiated cell by using the ultrasonic generation device, the laser generation device, or the temperature control device and culturing the mixture for a predetermined time. In this case, the ultrasonic waves, the laser, or the heat treatment may be performed in advance for the culture medium before mixing the culture medium with the differentiated cell in order to increase reprogramming efficiency.

Further, the cell reprogramming apparatus 100 may include a humidity control device 130 for controlling the humidity of the culture chamber. The temperature, the humidity, and the carbon dioxide amount in the culture chamber 110 may be appropriately controlled through the temperature control device 120, the humidity control device 130, and the gas control device (carbon dioxide control unit) 140 described above.

Further, the cell reprogramming apparatus 100 may include a display unit 150 for controlling the temperature, the humidity, and the carbon dioxide amount. A user operates the display unit to control the temperature, the humidity, and the carbon dioxide amount in the culture chamber 110. The display unit 150 may include a touch input type or button input type input unit.

Further, the sample tube 160 accommodating the cells may be disposed in the ultrasonic chamber 201, and the ultrasonic generation device 200 may be provided to be elevatable toward the sample tube 160. In addition, the sample tube holder 170 for supporting the sample tube 160 may be provided in the ultrasonic chamber 201. Further, the sample tube 160 may have a dual tube structure. In addition, the sample tube holder 170 may have a temperature control function and, for example, may be provided as a heat block.

Specifically, the culture chamber 110 may include the sample tube 160 accommodating the cells to which the ultrasonic waves are applied. Further, the ultrasonic generation device 200 is disposed to generate the ultrasonic waves into the sample tube 160, and as a result, the sample tube holder 170 for supporting the sample tube 160 is provided in the ultrasonic chamber 201. Meanwhile, the ultrasonic generation device 200, specifically, the ultrasonic transducer 220 may be provided to be elevatable so as to correspond to input/output of the sample tube 160 and sample tubes 160 having various sizes. In addition, the display unit 150 may be operated to elevate or lower the ultrasonic generation device 200, particularly, the ultrasonic transducer 220.

Further, a transport unit 230 on which the ultrasonic transducer 220 is elevatably mounted may be provided so as to close an inlet of the sample tube 160 when lowered. In addition, in order to maintain temperature, humidity, and gas levels in the ultrasonic chamber 201, a lid 111 surrounding the ultrasonic generation device 200 and the sample tube 160 may be provided. The lid 111 may be mounted to be openable/closable and may be made of a transparent material such as glass or a transparent resin.

Referring to FIG. 3, in the ultrasonic generation device 200, various types of ultrasonic transducers 220-1, 220-2, and 220-3 may be mounted to be replaceable depending on the size of the sample tube and the radiation characteristics of the ultrasonic waves.

Referring to FIG. 4, the sample tube 160 may have the dual tube (161 and 162) structure. Further, the sample tube 160 may be inserted into the sample tube holder 170. In this case, the sample tube 160 may be made of a material which has excellent refractory and heat resistance (e.g., a resin material) and as the sample tube holder 170 surrounds the sample holder, a soundproof effect may be realized. Specifically, the sample tube holder 170 is preferably provided as a space sealed to prevent cell contamination and it is preferable that the sample tube holder 170 includes a soundproof function to block noise due to the ultrasonic waves. Meanwhile, the sample tube holder 170 may be provided to perform a function of the temperature control device (for example, the heat wire).

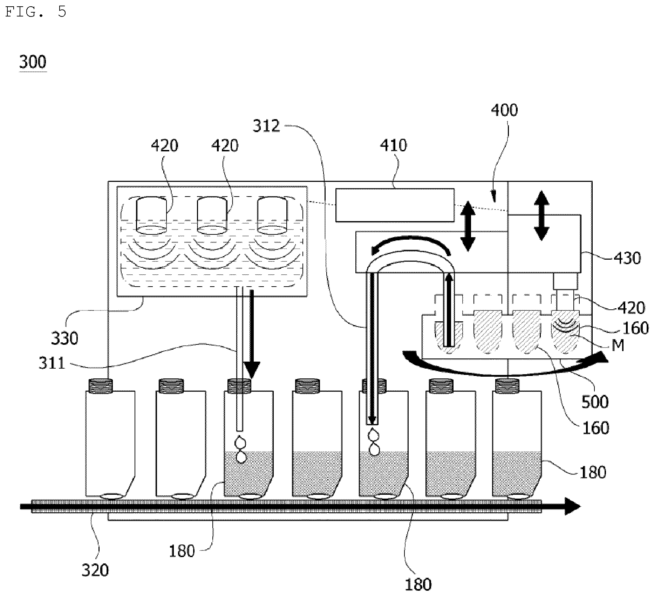

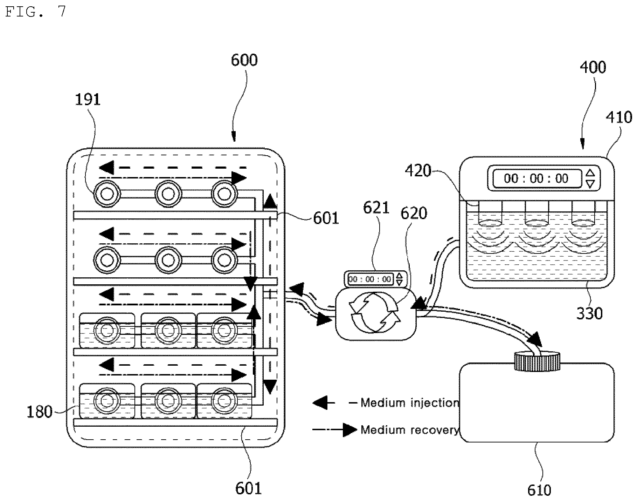

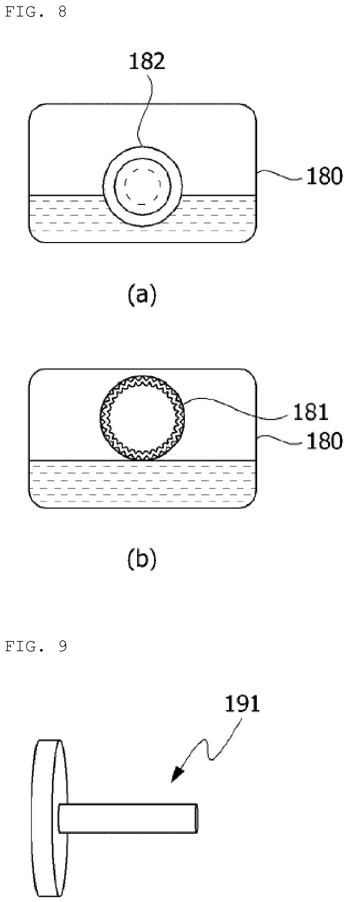

FIGS. 5 to 7 are conceptual diagrams illustrating a cell reprogramming apparatus 300 according to a second embodiment of the present invention, and FIGS. 8 to 10 are diagrams illustrating a culture flask 180 constituting the cell reprogramming apparatus 300.

Referring to FIG. 5 the cell reprogramming apparatus 100 described in FIGS. 1 to 4 may be applied to large-scale automated facilities.

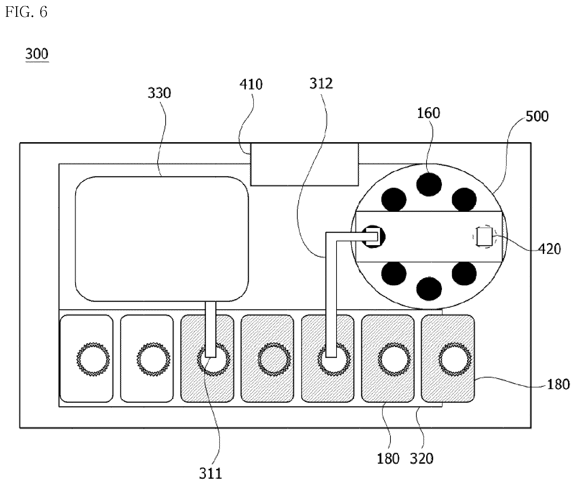

Referring to FIGS. 5 and 6, a cell reprogramming apparatus 300 according to a second embodiment of the present invention includes an auto sampler 500 provided so that a plurality of sample tubes 160 may be mounted and an ultrasonic generation device 400 provided so as to generate the ultrasonic waves to any one sample tube mounted on the auto sampler 500.

Further, the auto sampler 500 is rotatably provided. In addition, the ultrasonic generation device 400 includes an ultrasonic generator 410, an ultrasonic transducer 420, and a transport unit 430 similarly to the first embodiment. In this case, the ultrasonic transducer 420 may be elevatably provided.

Further, when the auto sampler 500 rotates, the ultrasonic generation device 400 may be in a elevated state.

In addition, the cell reprogramming apparatus 300 may additionally include a culture medium tank 330 storing a culture medium which is treated with ultrasonic waves, one or more culture flasks 180 to which the culture medium in the culture medium tank 330 is supplied, and an injection tube 312 connecting the culture flask 180 to the sample tube 160 for fluid flow.

Further, the cells in the sample tube 160 to which the ultrasonic waves are applied may be provided to move to the culture flask 180 by the injection tube 312.

In addition, the cell reprogramming apparatus 300 may additionally include a moving belt 320 for transporting a plurality of culture flasks 180.

Further, the cell reprogramming apparatus 300 may additionally include an incubator 600 accommodating the culture flask 180, a recovery tank 610 recovering the culture medium of the culture flask 180 in the incubator 600, and a circulator 620 for supplying the culture medium to the culture flask 180 in the incubator 600 and recovering the culture medium in the culture flask 180 to the recovery tank 610.

In addition, flow passages may be provided at both sides of the culture flask 180, respectively, and further, the flow passages at both sides of the culture flask may be positioned at different heights. Further, caps 181 and 182 may be mounted on the flow passages, respectively. In addition, an injector 191 for flowing of the culture medium may be provided to be mounted on at least one cap 182.

Specifically, the automatic cell reprogramming apparatus 300 according to the second embodiment includes an auto sampler on which a plurality of sample tubes may be mounted. The auto sampler is rotatably provided.

Further, the aforementioned ultrasonic generation device 400 is provided. Here, the ultrasonic generation device 400 is disposed to generate ultrasonic waves in any one sample tube mounted on the auto sampler 500. For example, the position of the ultrasonic generation device 400 may be fixed. Of course, as described above, the ultrasonic generation device 400 may be provided so as to be elevated and lowered in a height direction through the transport unit 430, etc.

For example, while the auto sampler 500 rotates, the ultrasonic generation device 400 (specifically, the ultrasonic transducer) may be in the elevated state and while the ultrasonic waves are generated to the positioned sample tube 160, the ultrasonic generation device 400 (specifically, the ultrasonic transducer) may be in a lowered state.

In this case, as the auto sampler 500 rotates, the plurality of sample tubes 160 moves to the ultrasonic transducer 420, and the ultrasonic transducer 420 generates ultrasonic waves to the positioned sample tube 160. That is, when the user mounts the plurality of sample tubes on the auto sampler 500, the respective sample tubes 160 are sequentially moved to the ultrasonic transducer 420 by the auto sampler 500 and ultrasonic waves may be applied to the cells in the sample tube 160.

Further, the cells to which ultrasonic waves are applied may be transported to the culture flask 180 through an injection tube 312 (also referred to as a second injection tube'). That is, while the auto sampler 500 rotates, ultrasonic waves may be applied to any one sample tube 160 and the cells in the sample tube 160 to which ultrasonic waves are applied may move to the culture flask 180 by the injection tube 312.

As described in the first embodiment, even in the automatic cell reprogramming apparatus 300 according to the second embodiment, internal temperature, gas, and humidities may be controlled and may be realized as an externally sealed apparatus (aseptic facility). Further, the use of the ultrasonic transducer 420 may also be alternated to a heat stick capable of applying the laser or heat shock.

In addition, a culture medium tank 330 storing the culture medium treated with ultrasonic waves is provided in the automatic cell reprogramming apparatus 300. The culture medium in the culture medium tank 330 is supplied to the culture flask 180 through a first injection tube 311. Meanwhile, the plurality of culture flasks 180 is transported to the incubator 600 from the auto sampler 500 by the moving unit such as the moving belt 320. Further, one or more ultrasonic transducers 420 are provided in the culture medium tank 330.

The automatic cell reprogramming apparatus 300 may additionally include a recovery tank 610 (waste tank) recovering the culture medium in the culture flask 180. In association with the ultrasonic treatment of the culture medium, the ultrasonic treatment apparatus 400 is provided in the culture medium tank 330 and generates ultrasonic waves to the culture medium in the culture medium tank 330 through one or more ultrasonic transducers 420. In this case, the display unit 150 (*see* FIG. 1) for setting and controlling the intensity of ultrasonic waves and the time may be provided. Further, a timer for counting the time during which the ultrasonic waves are applied may be provided.

In this case, the circulator 620 may be provided, which is used for supplying the culture medium to the culture flask 180 in the incubator 600 and recovering the culture medium in the culture flask 180 to the recovery tank 610 when a medium replacement time is reached. The circulator 620 is provided to recover the culture medium in the culture flask 180 to the recovery tank 610 when the medium replacement time set in the timer 621 has elapsed, and provided to supply the culture medium treated with ultrasonic waves by the ultrasonic transducer 420 from the culture medium tank 330 to the respective culture flasks 180 by means of the circulator 620.

Further, a shaker 601 for shaking the seated culture flask may be provided in the incubator 600. For example, the shaker 601 may be provided to shake the culture flask like a seesaw.

Meanwhile, the incubator 600 may set and control the internal temperature, gas, and humidities and may be realized as the externally sealed apparatus (aseptic facility) similarly to the culture chamber 110 described in the first embodiment.

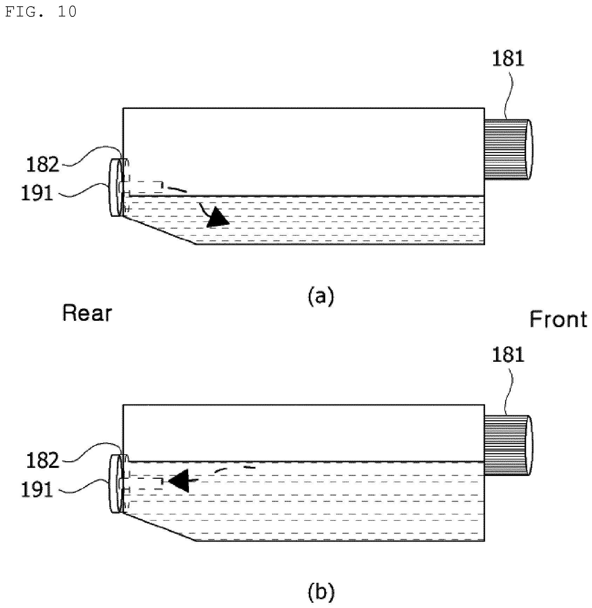

Referring to FIGS. 8 to 11, the culture flask 180 may have a structure capable of being docked on both sides so as to automatically inject and recover the medium at the injection tube and the incubator 600. Referring to FIGS. 8 and 10, the flow passages may be provided on both sides (e.g., front and rear) of the culture flask 180. Further, caps 181 and 182 made of rubber or a resin may be provided on the flow passages, respectively. In particular, as a docked portion is made of rubber, the docked portion may be provided so that the culture medium does not come out. Particularly, a height of the docked portion is adjusted so that only a proper amount may be recovered at the time of recovering the medium. For example, at the time of recovering the medium, the medium of up to the height of a rear flow passage may be recovered. Further, referring to FIGS. 9 and 10, at least one cap may be provided to be coupled with the injector 191 for the flowing of the culture medium.

The preferred exemplary embodiment of the present invention has been described above for illustrative purposes, and those skilled in the art will appreciate that various changes, modifications, and additions are possible within the scope and spirit of the present invention, and it shall be considered that the changes, modifications, and additions are included in the appending claims of the present invention.

### [Industrial Applicability]

The cell reprogramming apparatus according to an embodiment of the present invention has an effect of inducing reprogramming into new types of pluripotent cells having pluripotent characteristics or arbitrary differentiated cells having a different expression type from the differentiated cells by applying energy such as ultrasonic waves, laser, or heat treatment to the differentiated cells.

## Claims

1. A cell reprogramming apparatus comprising:
a culture chamber to accommodate cells and a culture medium; and
one or more devices to provide energy by applying at least one physical stimulation of ultrasonic waves, laser, and heat to the cells and the culture medium in the culture chamber.

2. The cell reprogramming apparatus of claim 1, wherein the devices include at least one of an ultrasonic generation device for irradiating the ultrasonic waves, a laser irradiation device for irradiating the laser, and a temperature control device.

3. The cell reprogramming apparatus of claim 2, further comprising:
at least one of a humidity control device for controlling humidity in the culture chamber and a gas control device for controlling a carbon dioxide amount in the culture chamber.

4. The cell reprogramming apparatus of claim 3, further comprising:
a display unit having an input unit for controlling at least one of a temperature, the humidity, and the carbon dioxide amount in the culture chamber.

5. The cell reprogramming apparatus of claim 4, wherein the devices include the ultrasonic generation device, and the display unit is provided so as to set an ultrasonic frequency and time.

6. The cell reprogramming apparatus of claim 2, wherein a sample tube accommodating the cells is disposed in the culture chamber, and the ultrasonic generation device is elevatably provided toward the sample tube.

7. The cell reprogramming apparatus of claim 6, wherein a sample tube holder for supporting the sample tube is provided in the culture chamber.

8. The cell reprogramming apparatus of claim 6, wherein the sample tube has a dual tube structure.

9. The cell reprogramming apparatus of claim 2, wherein an ultrasonic transducer is replaceably provided in the ultrasonic generation device.

10. The cell reprogramming apparatus of claim 2, wherein the temperature control device includes a heat wire provided around the culture chamber.

11. A cell reprogramming apparatus comprising:
an auto sampler provided such that a plurality of sample tubes may be mounted; and
an ultrasonic generation device provided to generate ultrasonic waves to any one sample tube mounted on the auto sampler.

12. The cell reprogramming apparatus of claim 11, wherein the auto sampler is rotatably provided, and the ultrasonic generation device is elevatably provided.

13. The cell reprogramming apparatus of claim 12, wherein when the auto sampler rotates, the ultrasonic generation device is in an elevated state.

14. The cell reprogramming apparatus of claim 11, further comprising:
a culture medium tank storing a culture medium treated with ultrasonic waves;
one or more culture flasks to which the culture medium in the culture medium tank is supplied; and
an injection tube connecting the culture flask to the sample tube for fluid flow.

15. The cell reprogramming apparatus of claim 14, wherein cells in the sample tube to which the ultrasonic waves are applied is provided to move to the culture flask by the injection tube.

16. The cell reprogramming apparatus of claim 14, further comprising:
a moving belt for transporting a plurality of culture flasks.

17. The cell reprogramming apparatus of claim 15, further comprising:
an incubator accommodating the culture flask;
a recovery tank recovering the culture medium of the culture flask in the incubator; and
a circulator for supplying the culture medium to the culture flask in the incubator and recovering the culture medium in the culture flask to the recovery tank.

18. The cell reprogramming apparatus of claim 14, wherein flow passages are provided at both sides of the culture flask, respectively.

19. The cell reprogramming apparatus of claim 18, wherein the flow passages at both sides of the culture flask are positioned at different heights.

20. The cell reprogramming apparatus of claim 17, wherein a shaker for shaking the culture flasks is provided in the incubator.
